# EUROPÄISCHE PATENTSCHRIFT

(11) **EP 1 617 883 B1**
(45) Veröffentlichungstag und Bekanntmachung des Hinweises auf die Patenterteilung: **16.08.2006**
(21) Anmeldenummer: 04728337.9
(22) Anmeldetag: 20.04.2004
(51) Int. Cl.: A61L 27/30, A61L 27/06, A61F 2/12

(54) **WEICHTEILIMPLANTAT WIE BRUSTIMPLANTAT, WADENMUSKELPROTHESE ODER DERGLEICHEN**
SOFT TISSUE IMPLANT, FOR EXAMPLE BREAST IMPLANT, CALF MUSCLE PROSTHESIS AND THE LIKE
IMPLANT DE TISSU MOU, PAR EXEMPLE IMPLANT MAMMAIRE, PROTHESE DE MUSCLE DE MOLLET OU ANALOGUE

(30) Priorität: 28.04.2003 DE 20306637 U
(43) Veröffentlichungstag der Anmeldung: 25.01.2006
(73) Patentinhaber: GfE Medizintechnik GmbH, 90431 Nürnberg (DE)
(72) Erfinder: ZIMMERMANN, Hanngörg, 90574 Rosstal (DE); FRICKE, Helmut, 38536 Meinerseden-Ahnsen (DE)
(74) Vertreter: Hübner, Gerd
(86) Internationale Anmeldenummer: PCT/EP2004/004170
(87) Internationale Veröffentlichungsnummer: WO 2004/096308

(56) Entgegenhaltungen:
- EP-A- 0 897 997
- WO-A-02/051465
- DE-A- 19 506 188
- US-A- 4 772 285
- US-A- 4 828 561
- US-A- 5 716 404
- US-A- 5 922 024
- US-B1- 6 322 588

## Beschreibung

Die Erfindung betrifft ein Weichteilimplantat umfassend eine äußere, blasenartige Hülle aus flexiblem Kunststoffmaterial, insbesondere aus Silikon, und einer flüssigen bis viskosen Füllung in der Hülle.

Derartige Weichteilimplantate werden beispielsweise in Form von Brustimplantaten auf dem Gebiet der plastischen Chirurgie nach Brustamputationen oder in der kosmetischen Chirurgie zur Brustvergrößerung eingesetzt. Weitere Anwendungen für solche Weichteilimplantate sind Prothesen für die Wadenmuskulatur oder Wangen-, Nasen-, Po-, Hoden- oder Oberarmmuskel-Implantate.

Problematisch bei derartigen Weichteilimplantaten ist die Tatsache, dass der Körper versucht, das aufgrund des an der Oberfläche liegenden Kunststoffinaterials als Fremdkörper wahrgenommene Implantat durch eine Einkapselungsreaktion zu separieren. Dazu wird relativ hartes, starres Gewebe aus dem Körper abgeschieden, das zu einer zumindest teilweisen oder lokalen Verkapselung des Implantats führt. Auch entzündliche Reaktionen durch das eingesetzte Weichteilimplantat treten bisweilen auf.

Aus der US 6,322,588 A sind medizinische Vorrichtungen bekannt, die mit Körperflüssigkeiten eines Patienten in Kontakt kommen. Es handelt sich um implantierbare Prothesen mit einem biokompatiblen Metallüberzug.

Die US 4,828,561 A offenbart ein biokompatibles Brustimplantat, das eine entsprechende Beschichtung auf Silikonpolymer-Basis mittels Funktionalisierung der Silikonoberfläche des Implantats mit primären und sekundären Aminogruppen aufweist.

Der Erfindung liegt die Aufgabe zu Grunde, ein gattungsgemäßes Weichteilimplantat so auszubilden, dass es in seiner Verträglichkeit und Sicherheit verbessert wird.

Diese Aufgabe wird durch die im Anspruch 1 angegebenen Merkmale gelöst, wonach eine metallhaltige, körperverträgliche, durchgehende Beschichtung auf der Außenseite der Implantathülle aufgebracht ist.

Neben der metallhaltigen Beschichtung auf der Außenseite der Hülle ist eine ebenfalls metallhaltige, durchgehende Beschichtung zusätzlich auf der Innenseite der Hülle aufgebracht. Es wurde nämlich festgestellt, dass die Beschichtung nicht nur eine Körperverträglichkeit von ansonsten unverträglichen Kunststoffmaterialien gewährleistet, sondern gleichzeitig als Diffusionssperre für verschiedenste Ionen und Moleküle fungiert. Insoweit bewirkt die innen- und außenseitige Beschichtung auf der Hülle aus Kunststoffmaterial zum einen, dass die im Kunststoffmaterial regelmäßig enthaltenen Weichmacher nicht mit der Zeit aus dem Material entweichen können. Damit bleibt die Flexibilität des Kunststoffmaterials auch über längere Zeitdauer erhalten. Zum anderen verhindert die Beschichtung ein Austreten der Füllung aus der Hülle in den Körperinnenraum, die aus viskosem Silikongel, physiologischer Kochsalzlösung oder Wasser bestehen kann. Insoweit verbessert die insbesondere zweiseitige metallhaltige Beschichtung das Dichtheitsverhalten des Weichteilimplantates drastisch, sodass wieder vermehrt auf Silikongel als Füllung zurückgegriffen werden kann. Silikongel hat nämlich bei einer Leckage der Hülle und Austreten in den Körperinnenraum nachteilige gesundheitliche Folgen, was bei Wasser oder physiologischer Kochsalzlösung nicht der Fall ist. Silikongel wird von den Anwendern jedoch aufgrund seiner dem Körpergewebe ähnlicheren Konsistenz bevorzugt. Auch vermeidet Silikongel aufgrund seiner gegenüber Wasser oder Kochsalzlösung höheren Viskosität Knickschäden im Bereich von engen Rundungen der Hülle, wie beispielsweise am Übergang eines Brustimplantats von seiner Außenwölbung in die dem Körper zugewandten Basis.

Bei dieser Beschichtung handelt es sich vorzugsweise um eine Titan-haltige Beschichtung mit einer Dicke von kleiner 2 µm, vorzugsweise von 5 bis 700 nm. Diese Beschichtung ist flexibel, womit sie die Bewegungen der Hülle vollständig und ohne Ablösungserscheinungen mitmachen kann. Ferner ist sie haft- und reibfest auf dem die Hülle bildenden Kunststoffmaterial aufgebracht.

Die metallhaltige Beschichtung besteht bevorzugt aus einer Verbindung der Formel TiₐO_{b}C_{c}, wobei gilt
a = 0,025 bis 0,9,
b = 0,025 bis 0,7 und
c = 0,2 bis 0,9.
Ggf. können im Austausch zu den Titan-Anteilen auch Tantal, Niob, Silber, Zirkon und Hafnium eingesetzt werden. Als weitere Elemente in der Verbindung können ferner Stickstoff und Bor beinhaltet sein.

Schließlich können die beiden Beschichtungen auf der Innen- und Außenseite des Implantates glatt oder rau ausgebildet sein, sodass die Oberflächenbeschaffenheit des Implantats unabhängig von der eigentlichen Hülle eingestellt werden kann.

Ein Ausführungsbeispiel der Erfindung wird anhand der beigefügten Zeichnung näher erläutert. Es zeigen:
- Fig. 1: einen schematischen Radialschnitt durch ein Brustimplantat, und
- Fig. 2: einen vergrößerten , schematischen Detailschnitt der Einzelheit II gemäß Fig. 1.

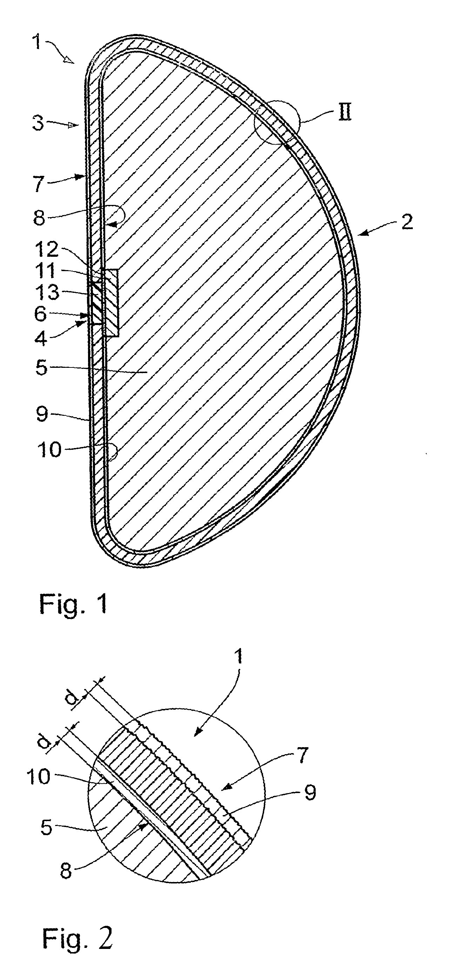

Wie aus Fig. 1 deutlich wird, weist das gezeigte Brustimplantat eine der natürlichen Brustform nachempfundene, äußere, blasenartige Hülle 1 auf, die aus flexiblem Silikonmaterial gefertigt ist. Die Hülle 1 setzt sich dabei aus einem in dem Körper abgewandten Richtung vorgewölbten Bereich 2 und einen im Wesentlichen ebenen, runden Basisbereich 3 zusammen. In diesem Basisbereich 3 ist zentral eine Öffnung 4 vorgesehen, die nach Einbringen der Füllung 5 in die Hülle 1 durch ein zweiteiliges Siegel 6 dicht verschlossen wird. Ein inneres Siegel 11 wird dabei über einen Klebering 12 an der Innenseite 8 der Hülle 1 um die Öffnung 4 herum angeklebt. Die außen verbleibende Vertiefung wird dann durch einen äußeren Siegeleinsatz 13 verschlossen. Die Füllung 5 besteht aus einem viskosen Silikongel und füllt den gesamten Innenraum der Hülle 1 so aus, dass diese zwar nicht prall, aber ohne Einfallstellen gefüllt ist.

Wie aus Fig. 2 deutlich wird, sind Außen- und Innenseite 7, 8 der Hülle 1 mit einer durchgehenden, körperverträglichen Beschichtung 9, 10 aus einem Titan-haltigen Material der Formel TiₐO_{b}C_{c} hergestellt. Die Anteilsbereiche a, b und c entsprechen denen in der Beschreibungseinleitung. Eine derartige Beschichtung hat sich als absolut körperverträglich herausgestellt. Durch ihre Durchgängigkeit wird die gesamte Hülle 1 mit ihrem Kunststoffmaterial nicht mehr als solches vom Körper wahrgenommen. Die Körperverträglichkeit ist somit mit komplett aus einer Titanlegierung gefertigten Implantaten vergleichbar, die in der Medizintechnik breite Akzeptanz fmden.

Bevorzugte Dicken d der Beschichtungen 9, 10 liegen im Bereich von 5 bis 700 nm, wobei Schichtdicken von etwa 50 nm sich als einerseits besonders haft- und reibfest, andererseits aber auch genügend flexibel und duktil herausgestellt haben, um alle Dehnungen und Verformungen der Hülle schadlos mit zu vollführen.

Derartige Titan-haltige Beschichtungen und die Technik zum Aufbringen auf flexible Kunststoffsubstrate sind im Übrigen aus dem Stand der Technik, wie etwa der EP 0 897 997 B1, grundsätzlich bekannt.

Wie ferner in Fig. 2 angedeutet ist, ist die Außenseite 7 der Hülle 1 an ihrer Substrat-Oberfläche leicht rau ausgebildet. Diese textuierte Oberfläche sorgt für eine Minimierung der Gefahr einer Kapselfibnose.

## Patentansprüche

1. Weichteilimplantat, wie Brustimplantat, Wadenmuskelprothese und dergleichen, umfassend
- eine äußere, blasenartige Hülle (1) aus flexiblem Kunststoffmaterial, insbesondere aus Silikon,
- eine flüssige bis viskose Füllung (5) in der Hülle (1), und
- eine metallhaltige, körperverträgliche, durchgehende Beschichtung (9) auf der Außenseite (7) der Hülle (1),
**dadurch gekennzeichnet, dass** eine metallhaltige, durchgehende Beschichtung (10) zuzätzlich auf der Innerseite (8) der Hülle (1) aufgebracht ist.

2. Weichteilimplantat nach Anspruch 1, **dadurch gekennzeichnet, dass** die Beschichtung (9, 10) eine Titan-haltige Beschichtung mit einer Dicke von kleiner 2 µm, vorzugsweise von 5 bis 700 nm ist.

3. Weichteilimplantat nach Anspruch 2, **dadurch gekennzeichnet, dass** die Beschichtung (9, 10) eine Verbindung der Formel
TiₐO_{b}C_{c}
aufweist, wobei gilt a = 0,025 bis 0,9
b = 0,025 bis 0,7 und
c = 0,2 bis 0,9.

4. Weichteilimplantat nach einem der vorgenannten Ansprüche, **dadurch gekennzeichnet, dass** die Außenseite (7) der Hülle (1) glatt ist.

5. Weichteilimplantat nach einem der Ansprüche 1 bis 3, **dadurch gekennzeichnet, dass** die Außenseite (7) der Hülle (1) an ihrer Oberfläche rau ist.

6. Weichteilimplantat nach einem der vorgenannten Ansprüche, **dadurch gekennzeichnet, dass** die Füllung (5) aus viskosem Silikongel besteht.

7. Weichteilimplantat nach einem der Ansprüche 1 bis 5, **dadurch gekennzeichnet, dass** die Füllung (5) aus physiologischer Kochsalzlösung oder Wasser besteht.

## Claims

1. A soft tissue implant, such as a breast implant, calf-muscle prosthesis and the like, comprising
- an external, shell-type envelope (1) of flexible plastic material, in particular silicone;
- a liquid to viscous filler material (5) contained in the envelope (1); and
- a metal-containing, biocompatible, continuous coating (9) on the outside (7) of the envelope (1);
**characterized in that** a metal-containing, continuous coating (10) is additionally applied to the inside (8) of the envelope (1).

2. A soft tissue implant according to claim 1, **characterized in that** the coating (9, 10) is a titanium-containing coating of a thickness of less than 2 µm, preferably of 5 to 700 nm.

3. A soft tissue implant according to claim 2, **characterized in that** the coating (9, 10) comprises a compound of the formula
TiₐO_{b}C_{c},
with a = 0.025 to 0.9,
b = 0.025 to 0.7 and
c = 0.2 to 0.9
applying.

4. A soft tissue implant according to one of the preceding claims, **characterized in that** the outside (7) of the envelope (1) is smooth.

5. A soft tissue implant according to one of claims 1 to 3, **characterized in that** the outside (7) of the envelope (1) has a rough surface.

6. A soft tissue implant according to one of the preceding claims, **characterized in that** the filler material (5) is viscous silicone gel.

7. A soft tissue implant according to one of claims 1 to 5, **characterized in that** the filler material (5) is a physiological sodium chloride solution or water.

## Revendications

1. Implant de tissu mou, par exemple implant mammaire, prothèse de muscle de mollet ou analogue, comprenant :
- une enveloppe extérieure gonflable (1) en matière plastique souple, plus particulièrement en silicone,
- un produit de remplissage allant de liquide à visqueux (5) dans l'enveloppe (1), et
- un revêtement à base de métal, compatible avec le corps et formé d'un seul tenant (9) sur la face externe (7) de l'enveloppe (1),
**caractérisé en ce qu'**un revêtement à base de métal formé d'un seul tenant (10) est appliqué en outre sur la face interne (8) de l'enveloppe (1).

2. Implant de tissu mou selon la revendication 1, **caractérisé en ce que** le revêtement (9, 10) est un revêtement à base de titane, d'une épaisseur inférieure à 2 µm, de préférence de 5 à 700 nm.

3. Implant de tissu mou selon la revendication 2, **caractérisé en ce que** le revêtement (9, 10) comporte une liaison ayant la formule
TiₐO_{b}C_{c},
dans laquelle
a = 0,025 à 0,9,
b = 0,025 à 0,7 et
c = 0,2 à 0,9.

4. Implant de tissu mou selon l'une des revendications précédentes, **caractérisé en ce que** la face externe (7) de l'enveloppe (1) est lisse.

5. Implant de tissu mou selon l'une des revendications 1 à 3, **caractérisé en ce que** la face externe (7) de l'enveloppe (1) est rugueuse en surface.

6. Implant de tissu mou selon l'une des revendications précédentes, **caractérisé en ce que** le produit de remplissage (5) est constitué de gel de silicone visqueux.

7. Implant de tissu mou selon l'une des revendications 1 à 5, **caractérisé en ce que** le produit de remplissage (5) est constitué de sérum physiologique ou d'eau.
